# EUROPEAN PATENT SPECIFICATION

(11) **EP 4 099 626 B1**
(45) Date of publication and mention of the grant of the patent: **18.02.2026**
(21) Application number: 22176802.1
(22) Date of filing: 01.06.2022
(51) Int. Cl.: H04L 9/40, G06F 21/64, G16H 10/40

(54) **METHOD, DEVICE AND COMPUTER PROGRAM FOR AUTHENTICATING A DATA SET**
VERFAHREN, VORRICHTUNG UND COMPUTERPROGRAMM ZUR AUTHENTIFIZIERUNG EINES DATENSATZES
PROCÉDÉ, DISPOSITIF ET PROGRAMME INFORMATIQUE POUR L'AUTHENTIFICATION D'UN ENSEMBLE DE DONNÉES

(30) Priority: 02.06.2021 EP 21177463
(43) Date of publication of application: 07.12.2022
(73) Proprietor: Abberior Instruments GmbH, 37077 Göttingen (DE)
(72) Inventor: DONNERT, Gerald, 37077 Göttingen (DE); KASTRUP, Lars, 37073 Göttingen (DE)

(56) References cited:
- WO-A1-2020/225488
- US-A1- 2019 180 850
- US-A1- 2020 351 094

## Description

### TECHNICAL AREA OF THE INVENTION

The invention relates to a method, a device and a computer program for authenticating a data set acquired from a specimen by a physical measuring instrument, in particular to prove that the data set is the original data set acquired by the physical measuring instrument, that the data set was generated by a certain user of the physical measuring instrument, and/or that the data set was acquired by a certain physical measuring instrument and/or that the original data set was acquired from a certain specimen. In particular, the data set may comprise image data obtained by an imaging device.

### PRIOR ART

In the age of growing digitalization and connectivity, data security becomes more and more important, as data theft and forging/tampering with electronic data increases.

In the area of the physical sciences, vast amounts of data are obtained by physical measuring instruments of various types (e.g., imaging devices) and published in electronic form.

Over the past years, there has been a growing tendency among scientists to publish data in shorter time intervals, e.g., using pre-print servers, which publish the data prior to a peer review process, or even in online social networks. It is hoped that this trend will lead to scientific discussions among a larger community, which may fuel the overall progress of science.

In spite of various advantages, the availability of scientific data in electronic, often publicly available, form increases the danger of data theft.

Moreover, manipulation of scientific data, especially image data (e.g., microscopic data), is relatively easy, and often hard to trace. There have been cases of forging scientific data which understandably have prompted the scientific community to become more suspicious in view of authenticity of scientific data. Furthermore, especially in image data, unintentional errors in data processing may generate processing artefacts leading to wrong scientific conclusions. Such errors are very difficult to trace, since the editing process is sometimes incompletely documented.

Being able to prove data authenticity is equally important in medical data obtained by physical measuring instruments, such as imaging devices. In addition, maintaining the privacy of patient data is of utmost importance in this field.

Thus, there is a need for methods of authenticating electronic scientific and medical data.

Furthermore, being able to trace the origin of data from physical measuring instruments is equally important in many other fields outside of science, e.g., in quality control of industrial products, in pathology or in forensics.

Authentication by digital signatures and digital certificates is common practice in electronic data communication. For example, in email communication, a digital signature can be generated by applying a hash function to the email content using a private key of the sender coupled to a public key. The recipient is able to verify the identity of the sender using the public key, e.g., with the help of a digital certificate issued by a trusted authority that links the public key to the sender.

The block chain technology was first introduced to implement the crypto currency 'bitcoin', but its use has recently been expanded to various technical applications involving data security.

A distributed ledger is a decentrally organized data structure implementing a transaction data base, e.g., a block chain or an acyclic graph.

A block chain (an example of a 'distributed ledger') is a sequence of interlinked data blocks which are typically stored in a distributed peer-to-peer computer network. Every block chain operates according to a defined set of rules allowing certain transactions. For a crypto currency such as 'bitcoin', these transactions typically involve creation of new fungible tokens (exchangeable currency units) and payments, i.e., transfer of the tokens between so-called electronic wallets of different users. Every transaction within the block chain must be verified and included in a new block, wherein each block typically includes a large number of transactions. E.g., according to the proof-of-work consensus mechanism, a new block is distributed over the peer-to-peer network storing the block chain, and the computers involved in the network apply a computationally expensive hash function using a hash value of the involved transactions as an input and attempt to acquire a specific result, e.g., a target number below a threshold (termed difficulty value) to verify the block. Any manipulation of transactions would change the outcome of the hash function and resulting false blocks would not be verified by the network. Since the hash value of the previous block is listed in each block, any manipulation affects all subsequent blocks and thus can be traced back to the block, where the error was introduced. In this manner, transactions in the block chain can be securely stored in a virtually non-changeable and non-erasable fashion.

In addition to fungible tokens (i.e., tokens which are indistinguishable from each other, have an equal value and are freely exchangeable, such as electronic currencies), block chain based non-fungible tokens (NFTs), which are unique or available in a restricted quantity have been implemented, e.g., in the Ethereum block chain. NFTs are generated by a transaction stored in the block chain, particularly, e.g., when using the Ethereum platform, by generating a smart contract with a unique owner identifier associated to the electronic wallet of a user. After generation of the token, a change of ownership is possible, so that NFTs can be traded for money, e.g., as representations of collectibles (digital trading cards, characters or items of online games) or representations of digital artworks.

An NFT associated to a certain wallet can be used as a manipulation-proof verification of ownership of a physical item or virtual commodity (such as a digital artwork).

Some methods for authenticating valuable physical items, such as paintings or gems, involving or referencing cryptography, particularly the block chain technology, are known from the prior art.

For example, WO 2019/232536 A1 describes a method for verifying the authenticity of precious gems. According to the disclosed method, an identifier, such as a number, a barcode or a QR code, is physically inscribed into the gem using short laser pulses. A non-fungible token (NFT) comprising a public key representing the identifier is then generated and stored in a block chain. Before a transaction involving the gem, the identifier can be optically read out and used to determine the corresponding public key stored in the block chain. The owner of the NFT can prove his or her identity (and hence his or her ownership of the gem) by providing the private key matching the public key.

Furthermore, international patent publication WO 2019/014284 A1 describes a method for verifying the authenticity of a painting, wherein the painting is illuminated by a modifiable light pattern via an array of LEDs, and a low-resolution microscopic image of the surface of the painting is captured by an array of CCD cameras. A set of cryptographic keys is generated from the image, e.g., using a wavelet transformation, and the keys are split into challenge-response pairs, wherein a challenge key represents a specific illumination pattern and the corresponding response key represents features of the image acquired when the painting is illuminated by the respective illumination pattern. The authenticity of the painting can later be verified using the same or a different imaging platform, by receiving a challenge key, illuminating the specimen by the respective illumination pattern, acquiring the image and calculating the response key. If the determined response key matches the challenge key, the inspected painting is verified to be the original item. According to an additional level of security, the challenge and response keys may be stored in a block chain. Notably, the described method addresses the authentication of a physical specimen itself, and not an authentication of the original dataset obtained from the specimen. This is an important difference, since in particular the generation of the cryptographic keys is chosen such that small changes between the original image and the image acquired during verification of authenticity still generate the same response key. This is crucial in the described context to account, e.g., for aging of the painting or small differences in the experimental setup. Therefore, the described method is not suitable to verify the authenticity of, e.g., a microscopic data set.

US patent application US 2020/0293500 A1 discloses a block chain based smart contract involving loan transactions of tractors. The method involves periodically obtaining images of a tractor and storing the image files in the block chain to track the condition of the tractor during the loan period. The subsequently submitted images are automatically compared to the original by image processing algorithms in a separate computer network, and upon detecting changes indicating damage to the tractor, the owner of the tractor may be notified, and penalties may automatically be executed by the smart contract. The disclosed block chain storage of the image data securely verifies the condition of the physical item (loan object) over time but is unable to verify the authenticity of the image data. Documents US 2020/351094, US 2019/180850 and WO 2020/225488 disclose relevant background art.

### OBJECTIVE OF THE INVENTION

In view of the prior art discussed above, the objective of the invention is to provide a method for authenticating a dataset obtained by a physical measuring instrument, particularly an imaging device, more particularly a microscope or a tomograph.

### SOLUTION

This objective is attained by the subject matter of the independent claims 1 (method), 11 (device) and 13 (computer program) and the aspects described hereafter. Embodiments of the invention are specified in sub claims 2 to 10 and 12 and are described hereafter.

### DESCRIPTION OF THE INVENTION

A first aspect of the invention relates to a method for authenticating a data set, wherein an original data set is acquired (particularly by a first user) from a specimen, particularly a specimen held by a specimen holder, using a physical measuring instrument. In particular, the physical measuring instrument is an imaging device, more particularly a microscope or a tomograph, even more particularly a microscope.

In the context of the present specification, the term 'physical measuring instrument' describes a device capable of determining a physical quantity of a specimen.

In the context of the present specification, the term 'imaging device' describes a device, particularly an optical device, capable of generating an image from a specimen (particularly held by the specimen holder) or localizing, that is obtaining the position in one to three dimensions in space, of at least one light emitter or light reflector (i.e., a fluorophore or a quantum bead) in the specimen. The imaging device comprises a wave source for generating waves (e.g., light or ultrasound) interacting with the specimen, and a detector for detecting a response of the specimen to the waves. The response may be a further wave emitted by the specimen.

In certain embodiments, the physical measuring instrument, particularly the imaging device, comprises a scanning device configured to direct the wave generated by the wave source to selected positions in the specimen, such that the wave interacts with a certain area or volume of the specimen. In particular, the scanning device is further configured to direct the response from a certain area or volume of the specimen to the detector. In case of an optical imaging device, such as an optical scanning microscope, e.g., the scanning device may be a galvanometric scanner which scans excitation light through the specimen and de-scans fluorescence light from fluorophores in the specimen to the detector. In particular, in case of optical imaging devices, such as microscopes and optical tomographs, the wave source is a light source for illuminating the specimen, and the detector may be a camera or a point detector such as a photomultiplier, a photodiode or a hybrid detector. However, the wave source may also generate electromagnetic waves of wavelengths outside of the visible spectrum, e.g., X-rays (in which case the imaging device may be a computer tomograph), or radio frequency waves coupled with magnetic fields as used, e.g., in magnetic resonance imaging. Furthermore, the wave source may emit other types of waves, such as acoustic waves, in which case the imaging device may be an ultrasound imaging device, particularly an ultrasound microscope. As used throughout this specification, the term 'microscope' describes an optical device which is configured to generate a magnified image of a part of the specimen or configured to localize light emitters or light reflectors (i.e., molecules or particles emitting or reflecting light) in the specimen. Examples of microscopes capable of generating magnified images are bright field microscopes, dark field microscopes, phase contrast microscopes, fluorescence microscopes and/or STED (stimulated emission depletion) or RESOLFT (Reversible Saturable Optical Linear Fluorescence Transitions) microscopes. Depending on how the image is generated, such microscopes may further be categorized into wide field microscopes and scanning microscopes (e.g., scanning confocal fluorescence microscopes). For instance, microscopes configured to localize light emitters or reflectors may be PALM/STORM microscopes or MINFLUX microscopes (see, e.g., F. Balzarotti et al.: "Nanometer resolution imaging and tracking of fluorescent molecules with minimal photon fluxes", Science 355, 606-612, 2017). These techniques determine the location of light emitters (e.g., fluorophores) or reflectors in the specimen with high precision. By localizing several emitters or reflectors, a high-resolution image of the sample can then be generated using computer algorithms.

In certain embodiments, the physical measuring instrument is an optical microscope, particularly a confocal microscope, a wide field microscope, a near field microscope, a STED microscope, a RESOLFT microscope, a MINFLUX microscope, a light sheet microscope, a screening microscope (i.e., a microscope configured to automatically screen compounds or cells) or a spinning disk microscope.

In certain embodiments, the physical measuring instrument is an electron microscope, particularly a transmission electron microscope, a scanning electron microscope or an electron tomograph. In particular, the electron microscope is a cryo electron microscope.

In certain embodiments, the physical measuring instrument is an atomic force microscope.

In certain embodiments, the physical measuring instrument is a tomograph, particularly an optical tomograph, an electron tomograph, an X-ray tomograph (i.e., a so-called 'computer tomograph') or a magnetic resonance imaging device (which may also be referred to as a magnetic resonance imaging scanner or a magnetic resonance tomograph).

In the context of the present specification, the term 'tomograph' describes an imaging device configured to obtain an image of the specimen in a layer-wise manner.

In certain embodiments, the physical measuring instrument is an ultrasound imaging device, particularly an ultrasound microscope (also referred to as a scanning acoustic microscope or an acoustic microimaging device).

The specimen may be a physical object, particularly having a microstructure in the size range between 1 nm and 500 µm. For example, the specimen may be a biological sample (e.g., comprising biological cells or tissue obtained from an organism or entire microorganisms). Alternatively, e.g., the specimen may be an industrial product, such as a semiconductor wafer. Yet alternatively, the specimen may be a part of a living or non-living organism, or even an entire living or non-living organism, such as an animal or a human.

Depending on the type of specimen, the specimen holder may take various forms. For instance, in case of a microscope, the specimen holder may be a holder for a microscope slide which carries the specimen, e.g., in an embedding medium under a coverslip. In case of a magnetic resonance imaging device, e.g., the specimen holder may be a table on which a patient rests during a magnetic resonance imaging session. A further example of a specimen holder of an ophthalmological imaging device could be a support keeping the head of a patient in a stable position while being examined.

In particular, obtaining the original data set from the specimen by the physical measuring instrument (particularly the microscope) particularly comprises obtaining at least one image by the physical measuring instrument (particularly the microscope) or obtaining the position of (i.e., localizing) at least one emitter or reflector in the specimen by the physical measuring instrument (particularly the microscope), generating a data representation of the image or a data representation of the at least one obtained emitter or reflector position by the first processor, and storing the data set comprising the data representation in a memory. For example, the data representation may comprise a plurality of pixels or voxels having a spatial coordinate and an intensity value (e.g., on a grey scale) representing a light intensity of the light from the specimen captured by the image or emitted by the localized at least one emitter or reflector at the corresponding spatial coordinate. Furthermore, in particular, the pixels or voxels may comprise a color value representing a wavelength of the light captured by the image or the light emitted by the at least one emitter or reflector.

The data set obtained by the physical measuring instrument is termed 'original data set' herein merely to distinguish the term from further data sets which are subsequently obtained from the original data set by data processing (yielding one or more processed data sets).

In case the original data set comprises more than one image, the images may, e.g., constitute a temporal series (e.g., a movie), a z-stack (a series of images obtained while moving the focus position in an axial direction parallel to an optical axis, along which the specimen is illuminated) or a tiled and/or stitched image (a larger field of view obtained by a combination of a plurality of images acquired from the same focal plane) or a combination thereof.

In certain embodiments, the original data set comprises content data and/or metadata. The content data are particularly image data (obtained from an image) or localization data (obtained by localizing at least one emitter or reflector). The metadata particularly indicate at least one measuring parameter of the physical measuring instrument used during acquisition of the original data set.

In particular, the metadata comprise data representing a time stamp, a GPS location, a system identifier of the physical measuring instrument, objective parameters (relating to a microscope objective), an indication of light sources, particularly laser sources used to illuminate the specimen (e.g., excitation, STED and/or activation lasers), a wavelength, a laser power (particularly an absolute laser power), a repetition rate or a pulse train of the laser sources (in case of pulse lasers), a setting of a spatial light modulator, a detector type or a setting (e.g., gating parameters, pinhole size) of a detector used to collect light from the specimen, a type or a setting of an optical filter, settings of a scanner, e.g., a galvanometric scanner, an electrooptic deflector or an acousto optic deflector, settings of a deformable mirror, a type of the specimen, a type of fluorophore used to label the specimen, a buffer and/or an embedding medium used to prepare the specimen and/or a temperature.

The method comprises generating, particularly by a first processor connected to or comprised in the microscope, a digital proof of authenticity indicating the authenticity of the original data set.

A processor 'connected to the physical measuring instrument' is particularly connected to the physical measuring instrument via an electric connection and/or a data connection, which transfers data between the physical measuring instrument and the processor as an electric signal, optical signal (e.g., by a fiber-optic cable) or by a signal transmitted in a wireless fashion by electromagnetic waves. There may be a direct connection between the physical measuring instrument and the processor (e.g., the physical measuring instrument and the processor may be located in the same building, particularly in the same room). Alternatively, the connection between the physical measuring instrument and the processor may be a remote connection (e.g., the processor may be implemented on a remote server, e.g., a cloud server).

In the context of the present specification, the term 'digital proof of authenticity' describes a data structure stored in at least one memory. The digital proof of authenticity indicates, with a high confidence level, the authenticity of the original data set. In other words, the digital proof of authenticity indicates that the original data set has not been altered, was generated and/or verified by a certain person (e.g., a researcher or technician operating the physical measuring instrument) and/or, was obtained by a certain physical measuring instrument (characterized, e.g., by a type and a serial number) and/or was obtained from a certain specimen. In particular, the digital proof of authenticity is configured such that the authenticity of the original data set can be verified by a cryptographic procedure.

Using the digital proof of authenticity, a data set, e.g., comprising a microscopic image, the origin of the data set can be verified with high certainty. Since the generation of the digital proof of authenticity is linked to the acquisition of the data set by the physical measuring instrument, the digital proof of authenticity further indicates with high confidence that the data have not been processed or manipulated. This can be used advantageously, e.g., during publishing of the data set. For instance, if a reference to the digital proof of authenticity is published along with the data set, a public user, a peer reviewer or an editor of a scientific journal can verify that the published data represent the original data and that the researcher claiming a scientific discovery rightfully obtained the data. In addition, in case the original data set comprises metadata, the conditions under which the data were obtained, e.g., a time stamp and physical measuring instrument settings, can be traced in a secure manner with a low probability of manipulations.

In certain embodiments, the digital proof of authenticity is automatically generated by the first processor in response to an original data set being acquired. In this manner, it can be ensured that all data obtained by the physical measuring instrument receive a digital proof of authenticity. For example, this automatic generation procedure may be activated by a user by altering a setting, e.g., in a graphical user interface (GUI), such as by checking a box or activating a slider in the GUI.

In certain embodiments, the digital proof of authenticity is generated by the first processor for an acquired original data set in response to a command received by a user, e.g., via a GUI (e.g., by clicking a button in the GUI). Thereby, only certain, user-defined data (e.g., those intended for publication) are tagged with a digital proof of authenticity, saving memory space and energy.

In certain embodiments, prior to generating the digital proof of authenticity, the original data set is displayed, e.g., in the physical measuring instrument GUI, in a protected manner, such that the original data cannot be modified, stored and/or exported to a freely displayable format. In particular, after generating the digital proof of authenticity, the original data set is displayed in a manner, such that the original data set can be processed (e.g., in the physical measuring instrument software) and/or exported to a freely displayable format (e.g., to process the data set in a separate software, extract data from the data set or save the data set in another format). In particular, upon receiving a user command indicating an attempt to modify and/or export the original data set prior to generating the digital proof of authenticity, a message may be displayed (e.g., as a text message in the GUI), wherein the message indicates that a digital proof of authenticity must be generated before the original data set may be processed, stored or exported. More particularly, a graphical item, e.g., a clickable button, configured to enter the command triggering generation of the digital proof of authenticity may be displayed, particularly in the GUI, along with the message.

In certain embodiments, identification of the first user and/or the physical measuring device is received by the first processor, wherein the identification is verified by the first processor, and wherein, in case the identification is valid, the digital proof of authenticity is generated by the first processor.

In certain embodiments, a user account assigned to the first user is stored in the first processor comprised in or connected to the physical measuring instrument, wherein the user account is accessible by a protected login procedure. The login procedure may be protected, e.g., by a password which the first user must enter. Alternatively, biometric data of the first user (e.g., a fingerprint or a retina scan) may be obtained by a biometric sensor and verified by the first processor before login is granted. Yet alternatively, a digital signature card verifying the identity of the first user may be connected to the physical measuring instrument or to the first processor.

In certain embodiments, the method according to the invention further comprises obtaining a first private key associated with the first user and/or a second private key associated with the physical measuring instrument and/or a third private key associated with the specimen, wherein particularly the digital proof of authenticity is generated using the first private key, and/or the second private key and/or the third private key. Therein, the term 'private key' describes a cryptographic key which is intended to be known only by the user identifying himself/herself with the private key (e.g., in case of the first private key by the first user) or a cryptographic key associated with a device and stored in a memory of the device or connected to the device in a manner, such that the cryptographic key is protected against unauthorized retrieval (e.g., in case of the second private key identifying the physical measuring instrument) or a cryptographic key associated with the specimen (e.g., in case of the third private key). In particular, the first private key and/or the second private and/or the third private key is/are linked to a corresponding public key (i.e., a cryptographic key intended to be revealed to the public to cryptographically verify the identify the user or the physical measuring instrument or the specimen). Therein, 'linked' particularly means that the private key and the corresponding public key have been generated together by a cryptographic algorithm.

In certain embodiments, the third private key is obtained by the first processor based on a physical identifier inscribed on or in the specimen or a specimen carrier carrying the specimen (e.g., a microscope slide or a cover slip covering the specimen). For example, in case the physical identifier is inscribed in the specimen itself, the physical identifier may be a pattern of fluorescent beads or light-reflecting particles, or any kind of pattern imprinted in an embedding medium in which the specimen is embedded, particularly such that it is readable by the physical measuring instrument. Furthermore, the physical identifier may be inscribed in a specimen carrier, e.g., by laser pulses, e.g., in a similar manner as described by US patent 9,370,956 B2. Alternatively, e.g., the physical identifier may be printed on a label attached to the specimen carrier. In particular, the third private key may be obtained by applying a hash function to a data representation of the physical identifier.

In certain embodiments, the third private key is determined based on a unique biological feature identifying the organism from which the specimen was obtained, or the organism constituting or comprised in the specimen, particularly a fingerprint, a retina structure or at least a partial DNA sequence. For example, the third private key may be obtained by applying a hash function to a data representation of the biological feature, e.g., an image of the fingerprint, a retina scan or a string representing the DNA sequence. In particular, the third private key may be obtained by applying a hash function to a data representation of the physical identifier of the specimen and a data representation of the biological feature. In this manner, the digital proof of authenticity points not only to the specimen, but also to the respective organism.

In particular, the first private key and/or the second private key and/or the third private key is/are stored in a memory comprised in the physical measuring instrument or directly connected to the physical measuring instrument. Therein, the first private key may be linked to the user account assigned to the first user owning the first private key. The first, second and/or third private key (and the fourth and fifth private key described below), particularly the second private key, may be stored, e.g., in a trusted platform module (TPM) in the first processor and/or the physical measuring instrument (see below).

In certain embodiments, the digital proof of authenticity is a non-fungible token (referred to hereafter by the acronym 'NFT') stored in a distributed ledger, particularly a block chain. In the context of the present specification, the term 'non-fungible token' describes a unique data structure stored in a distributed ledger, particularly in a block chain, wherein the data structure represents the original data set. As used throughout the present specification, the term 'block chain' describes a series of linked data blocks stored on a plurality of physical memories comprised in processors forming a distributed network. Each of the data blocks may comprise data representing at least one transaction, e.g., generation (also termed 'minting') of an NFT representing a specific original data set, modification/updating of such an NFT (see below) and/or or deletion (also termed 'burning') of such an NFT. Each of the blocks may comprise a header, a previous hash value, i.e., a hash value of the previous block in the block chain, a Merkle root representing a hash value of the data representing the transactions and/or a nonce. Each new block may be verified by the distributed network by a consensus mechanism, e.g., proof-of-work, proof-of-stake, proof-of-capacity or proof of burn. The distributed network may be a public network or a private network, e.g., by a trusted authority.

An advantage of an NFT as a digital proof of authenticity is that the data verifying the authenticity of the original data set can be stored in a virtually un-modifiable and un-erasable manner. If the data structure of the NFT should be manipulated after storing the NFT in the distributed ledger, this will alter the transactions and thus lead to a failure in verification and a branching of the distributed ledger, which can be traced and rectified with help of the consensus mechanism of the distributed network.

In certain embodiments, the NFT comprises a first content identifier identifying or encoding at least a part of the original data set, particularly at least a part of the content data and/or at least a part of the metadata of the original data set. In particular, the first content identifier is a hash value obtained by applying a hash function to the original data set, particularly to the content data and/or the metadata. E.g., in a manner known from the prior art, a hash function may be applied to the bits of an image data file characterized by an array of pixel or voxel intensity values. Likewise, a hash value from metadata of the original data set may be obtained. The content identifier requires a distributed ledger architecture, particularly a block chain architecture, allowing storage of data in NFTs. This is possible, e.g., for NFTs governed by smart contracts implemented by the ERC-721 standard in the Ethereum block chain.

The first content identifier is a unique identifier (e.g., a number or a string) of the content data of the original data set, e.g., the image file(s) or localization data of emitters or reflectors. If, e.g., a manipulated image derived from the original data set would yield (e.g., by applying the hash function) a content identifier different from the one stored for the NFT in the distributed ledger. Thereby, manipulation or forging of images could easily be detected using the first content identifier of the NFT as a secure reference.

Besides the content data, the first content identifier may further represent at least a part of the metadata of the original data set. For example, a first hash value may be obtained from the content data, a second hash value may be obtained from the metadata, and a third hash value may be obtained from the first hash value and the second hash value, wherein the third hash value is the first content identifier.

In certain embodiments, the NFT further comprises a first metadata identifier (in addition to the first content identifier) identifying or encoding at least a part of the metadata of the original data set. For example, the first metadata identifier may be generated by obtaining a hash value by applying a hash function to the metadata. A separate metadata identifier has the advantage that the authenticity of the metadata can be safely verified via the distributed ledger separately from the content data.

In certain embodiments, the NFT comprises a unique first owner identifier indicating the first user who is the owner/creator of the NFT. In particular, the first owner identifier is linked to the first private key associated with the first user. For example, the first owner identifier may be a public key linked to the first private key (that is generated, in a cryptographic procedure together with the first private key).

In certain embodiments, the NFT comprises a unique second owner identifier indicating a second user authenticating the original data. In particular, the second owner identifier is linked to a fourth private key associated with the second user authenticating the original data. For example, the second owner identifier may be a public key linked to the fourth private key.

In certain embodiments, the non-fungible token comprises a unique first device identifier indicating the physical measuring instrument. In particular, the first device identifier is linked to the second private key associated with the physical measuring instrument. For example, the first device identifier may be a public key linked to the second private key (that is generated, in a cryptographic procedure together with the second private key).

In certain embodiments, the non-fungible token comprises a unique specimen identifier indicating the specimen. The specimen identifier may be generated based on a physical identifier inscribed in the specimen or inscribed in a component of a specimen carrier carrying the specimen. The physical identifier may be inscribed in the specimen or the specimen carrier as described above. Such a specimen identifier has the advantage that not only information on the user and the physical measuring instrument, but also information on the specimen can be stored in a virtually non-modifiable and non-erasable manner in the blockchain. Since the data in the blockchain can be linked to a physical specimen, which could be re-examined (provided that the specimen is sufficiently long-lived), this further improves the chances to detect manipulations and resolve doubts about data authenticity. For example, the specimen identifier may be a public key linked to the third private key.

In certain embodiments, in case the specimen contains biological material, the specimen identifier may be determined based on a unique biological feature identifying the organism from which the specimen was obtained, or the organism constituting or comprised in the specimen. For example, such a biological feature may be a fingerprint, a retina structure or at least a partial DNA sequence. In this manner, the original data set is not only linked to the specimen from which it was obtained, but also to the organism providing the specimen.

In certain embodiments, the non-fungible token comprises a unique organism identifier which is determined based on a unique biological feature identifying the organism from which the specimen was obtained, or the organism constituting or comprised in the specimen, particularly a fingerprint, a retina structure or at least a partial DNA sequence.

In certain embodiments, the first owner identifier, the second owner identifier and/or the first device identifier and/or the specimen identifier are stored in a non-erasable and non-editable manner in the distributed ledger. In other words, the first and/or second owner identifier and/or the first device identifier and/or the specimen identifier can never be changed after generation of the NFT. This is in contrast to tradable NFTs according to the prior art, which can be transferred from one wallet to another, e.g., in exchange for money, whereby their owner identifier is changed. The NFT according to the present invention, however, is not meant to be traded, but serves to verify the identity of the person having generated a data set and/or the device with which the data set was obtained and/or the examined specimen. The unique and unchangeable owner and/or device and/or specimen identifier therefore contributes to safely verifying the authenticity of the original data set.

In certain embodiments, after acquiring the original data set by the physical measurement instrument, the first private key identifying the first user is obtained by the first processor comprised in or connected to the physical measuring instrument, particularly the imaging device, more particularly the microscope or tomograph, the second private key identifying the physical measuring instrument is obtained by the first processor, and an NFT authenticating the original data set is generated by the first processor using the first private key and the second private key.

In certain embodiments, the distributed ledger, particularly the block chain, is stored in a distributed manner in a peer-to-peer network, wherein particularly the first processor forms a full node of the peer-to-peer network. For example, users wishing to protect their data using NTFs according to the method described in the present specification may combine their efforts to maintain their own distributed ledger to mutually verify data security. Alternatively, the first processor may take part in a larger public distributed ledger.

In certain embodiments, the distributed ledger, particularly the block chain, is stored in a private network. This has the advantage that the network is better protected against potential attacks.

In certain embodiments, the distributed network, particularly the peer-to-peer network or the private network, comprises a plurality of full nodes determining a consensus on propagation of the distributed ledger, particularly the block chain.

In certain embodiments, consensus on propagation of the distributed ledger, particularly the block chain, is determined by proof-of-stake, proof-of-capacity, proof-of-burn or proof-of-activity. In other words, consensus is determined by a mechanism which is not proof-of-work. As known from the prior art, according to proof-of-work, the full nodes of the distributed ledger, particularly the block chain (miners) conduct complicated mathematical calculations, which require a high processing power and thus consume a lot of energy. For this reason, proof-of-work has been criticized for its unfavorable CO₂ footprint. Alternative consensus mechanisms are thus preferred, although proof-of-work is well-established and considered very safe.

In certain embodiments, at least one processed data set is generated from the original data set, wherein the digital proof of authenticity is updated, such that the digital proof of authenticity indicates the authenticity of the original data set and the at least one processed data set.

For instance, the original data set may be processed by an image-processing method, such as, e.g., de-convolution, contrast and/or brightness adjustment, filtering, background correction, binning or averaging of series of images, overlay of images from different channels and/or false coloring.

In this manner, representations of processed versions of the original data set can be safely stored in the distributed ledger, particularly the block chain, such that every processing step can be documented for later verification. Thereby, a high level of data transparency is achieved. For example, in case of scientific data, journal editors, peer reviewers or the scientific community may verify each processing step and analyze if the step was performed in a scientifically accepted manner without manipulation or involuntary introduction of processing artefacts.

In certain embodiments, the NFT further comprises at least one second content identifier identifying or encoding content data and/or metadata of the processed data set. In particular, a new second content identifier is generated each time the NFT is updated following a processing step.

By means of the at least one second content identifier, proof of authentication may be provided for any number of processed data sets derived from the original data set while maintaining the proof of authentication of the original data set.

In certain embodiments, the NFT comprises at least one second metadata identifier representing metadata related to the processing of the original data set yielding the processed data set. The metadata related to the processing may represent processing parameters. Examples of such processing parameters may include a filter type (e.g., Gaussian filter) of a filter applied to the original data set to generate the processed data set as well as filter parameters (e.g., a width of the Gaussian filter). By means of the second metadata identifier, the entire chain of processing events of the original data set can be made transparent and traceable.

In certain embodiments, the second content identifier is a hash value obtained by applying a hash function to the original data set and the processed data set. In particular, the first content identifier and the second content identifier are hash values, wherein the hash values are linked to each other. For example, a first hash value constituting the first content identifier may be generated from the original data set and a second hash value constituting the second content identifier may be generated from the processed data set and the first hash value.

In certain embodiments, the original data set is processed to obtain the processed data set on the same processor, on which the digital proof of authenticity is updated. This may be the first processor, which is comprised in or connected to the physical measuring instrument, and on which the original data set was obtained. Alternatively, after generating the digital proof of authentication from the original data set, the original dataset may be transferred to a second processor used for processing of the data (separate from the first processor comprised in or connected to the physical measuring instrument).

In certain embodiments, the digital proof of authenticity is automatically updated in response to processing the original data set. Alternatively, the digital proof of authenticity may be updated in response to a user command (e.g., clicking a button in a GUI).

In certain embodiments, a user account assigned to a user is stored in the second processor, wherein the user account is accessible by a protected login procedure (e.g., using a password, biometric data, and/or a digital signature card).

In certain embodiments, the first private key is obtained by the (first or second) processor updating the digital proof of authentication, wherein the digital proof of authenticity is updated using the first private key.

In certain embodiments, a fifth private key is obtained by the (first or second) processor processing the original data set to generate the processed data set, wherein particularly the digital proof of authenticity is updated using the fifth private key, particularly using the first private key and the fifth private key. In particular, the fifth private key may be stored in a trusted platform module of the first or second processor as described from the second private key or in a similar manner.

In certain embodiments, the digital proof of authentication is a digital signature.

In certain embodiments, the digital signature is generated using the first private key (identifying the first user) and/or the second private key (identifying the physical measuring instrument) and/or the third private key identifying the specimen, and a hash value obtained by applying a hash function to the original data, particularly to the content data and/or the metadata of the original data set.

In certain embodiments, the first private key and/or the second private key and/or the third private key is/are linked to a corresponding public key.

When the original data set is signed using the digital signature, the origin of the data can be verified using a public key linked to the first and/or second private key. **To** this end, e.g., a digital certificate verifying that the first private key linked to a certain public key is owned by the first user and/or that the second private key linked to a certain public key belongs to a certain physical measuring instrument and/or that the third private key linked to a certain public key belongs to a certain specimen. Such a certificate may be issued, e.g., by a trusted authority.

In certain embodiments, at least one processed data set is generated from the original data set, wherein the digital proof of authenticity, particularly the digital signature, is updated, such that the digital proof of authenticity, particularly the digital signature, indicates the authenticity of the original data set and the at least one processed data set.

In certain embodiments, the hash function is applied to the original data set, particularly to the content data and/or the metadata of the original data set and the processed data set to generate a combined hash value, wherein the digital proof of authenticity, particularly the digital signature, is updated using the combined hash value.

In certain embodiments, the combined hash value is generated by obtaining a first hash value by applying the hash function to the original data set and obtaining a second hash value by applying the hash function to the first hash value and the processed data set, particularly the content data and/or the metadata of the processed data set.

Alternatively, e.g., the processed data set may comprise metadata comprising the first hash value of the initial data set, and the combined hash value may be obtained from the processed data set using the metadata.

In certain embodiments, the digital proof of authenticity is automatically generated by the first processor in response to the original data set being acquired. In certain embodiments, the original data set comprises metadata, wherein at least a part of the metadata is automatically selected by the first processor, and wherein the digital proof of authenticity indicates the authenticity of the selected metadata. In particular, the metadata indicate at least one measuring parameter of the physical measuring instrument used during acquisition of the original data set.

In certain embodiments, the digital proof of authenticity is generated by the first processor for the acquired original data set in response to a user command received by the first processor. In certain embodiments, the original data set comprises metadata, wherein at least a part of the metadata is selected, wherein the digital proof of authenticity indicates the authenticity of the selected metadata. According to a first alternative, the metadata are automatically selected by the first processor. According to a second alternative, the metadata are selected based on a user selection received by the first processor. In particular, the metadata indicate at least one measuring parameter of the physical measuring instrument used during acquisition of the original data set.

In certain embodiments, additional metadata are generated by the first processor upon generation of the digital proof of authenticity in response to a user selection received by the first processor.

In certain embodiments, the original data set and/or the processed data set is uploaded, particularly from the first processor or the second processor, to a publication server to publish the original data set and/or the processed data set, particularly together with the digital proof of authenticity. In particular, the original data set or the processed data set is uploaded to the publication server in response to a user command, e.g., in response to clicking a button in a GUI.

A second aspect of the invention relates to a device for authenticating a data set, particularly by the method according to the first aspect, wherein the device comprises a physical measuring instrument, particularly an imaging device, more particularly a microscope or a tomograph, even more particularly a microscope, and, particularly, a specimen holder configured to hold a specimen. The physical measuring instrument is configured to acquire an original data set from the specimen, particularly the specimen held by the specimen holder. The device for authenticating a data set further comprises a first processor (which is particularly comprised in or connected to the physical measuring instrument) configured to generate a digital proof of authenticity indicating the authenticity of the original data set.

In certain embodiments, particularly in case the physical measuring instrument is a microscope, the physical measuring instrument comprises a light source for generating light to illuminate the specimen, particularly when the specimen is held by the specimen holder, an objective for focusing the light generated by the light source into the specimen, and a detector configured to detect light from the specimen.

In particular, the physical measuring instrument is a confocal scanning fluorescence microscope, particularly a STED or RESOLFT microscope, or a MINFLUX microscope.

In certain embodiments, the first processor is configured to obtain a first private key associated with the first user of the physical measuring instrument, and/or a second private key associated with the physical measuring instrument, and/or a third private key associated with the specimen, particularly via a user interface or from a memory or by reading a physical identifier inscribed in the specimen or a specimen carrier carrying the specimen. In particular, the first processor is configured to generate the digital proof of authenticity using the first private key and/or the second private key and/or the third private key.

In certain embodiments, the device, particularly the physical measuring instrument or the first processor, comprises a trusted platform module. In particular, the first private key, the second private key, the third private key, the fourth private key and/or the fifth private key, more particularly the second private key associated with the physical measuring instrument, is stored on the trusted platform module. In particular, the trusted platform module is configured to generate a hash value by applying a hash function at least to the second private key and/or generate the digital proof of authenticity. A trusted platform module improves the safety of the cryptographic keys involved in the generation of the digital proof of authenticity.

In the context of the present specification, the term 'trusted platform module' (TPM) describes a hardware component, such as a microprocessor chip, comprising a processing unit and at least one memory unit, particularly a volatile memory and/or a non-volatile memory, wherein the hardware component is configured to store at least one cryptographic key in the memory unit, particularly wherein the cryptographic key cannot be retrieved from the hardware component.

Optionally, the processing unit may comprise a random number generator, a cryptographic key generator, a hash generator, and/or an encryption/decryption unit. An endorsement key, a storage root key, a platform configuration register and/or an attestation identity key may be saved in the volatile and/or non-volatile memory (see the TCG specification published as standard ISO/IEC 11889:2015 for explanation of the terminology and as an example of a trusted platform module according to the invention).

In certain embodiments, the TPM is configured to store at least the second private key in the memory unit, particularly in a non-retrievable manner, wherein the TPM, particularly the processing unit of the TPM, more particularly the cryptographic key generator, is configured to derive at least one secondary key from the second private key. The digital proof of authenticity, particularly the NFT or the digital signature, may then be generated based on the at least one secondary key derived from the second private key.

A third aspect of the invention relates to a computer program comprising instructions configured to cause the device according to the second aspect to execute the method according to the first aspect.

Further embodiments of the device according to the second aspect and the computer program according to the third aspect have already been mentioned in the context of the method according to the first aspect.

Further embodiments of the invention may be derived from the claims, the description and the drawings. Therein, the claims are not to be construed in a manner such that only subjects, devices or methods respectively comprising all or none of the features of a sub claim in addition to the features of the independent claims and aspects described herein, may be possible embodiments of the invention. In contrast, further embodiments may be drawn from features stated in the description or derivable from the drawings which may be singly or cumulatively applied.

### SHORT DESCRIPTION OF THE FIGURES

The invention is further elucidated and described hereafter with reference to the exemplary embodiments displayed in the figures. These embodiments are non-restrictive examples which are not meant to limit the scope of the invention.
- **Fig. 1**: shows a schematic of a device according to an embodiment of the invention;
- **Fig. 2**: shows a flowchart illustrating the steps of a first embodiment of the method according to the invention using an NFT as digital proof of authentication;
- **Fig. 3**: shows a schematic of an example of an NFT used as digital proof of authentication according to the first embodiment of the method shown in Fig. 2;
- **Fig. 4**: shows a schematic of a block chain which can be used to store the NFT shown in Fig. 3;
- **Fig. 5**: shows a flowchart illustrating the steps of a second embodiment of the method according to the invention using a digital signature as digital proof of authentication;
- **Fig. 6**: shows a flowchart illustrating the steps of a verification procedure of the digital signature used in the second embodiment shown in Fig. 5.

### DESCRIPTION OF THE FIGURES

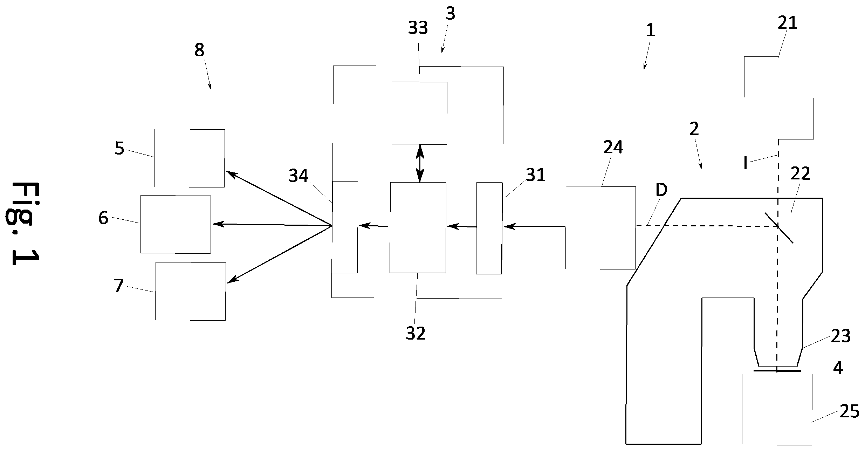

**Fig. 1** shows a device 1 for authenticating a data set according to an embodiment of the present invention. The device 1 comprises a physical measuring instrument 2 which is a microscope and a first processor 3 connected to the physical measuring instrument 2. The physical measuring instrument 2 comprises a light source 21 for generating illumination light I (e.g., a laser source). The illumination light I passes from the light source 21 through a beam splitter 22 (e.g., a dichroic mirror) and is focused by an objective 23 into a specimen 4 held by a specimen holder 25 of the physical measuring instrument 2. Detection light D (e.g., fluorescence light from fluorophores in the specimen 4 or reflected light from particles in the specimen 4) emanates from the specimen 4 and is separated from the illumination light I and reflected into a detection beam path by the beam splitter 22. The detection light D hits a detector 24 which converts the light into signals (e.g., electric signals). These signals are passed to an input 31 of the first processor 3 which is connected to the detector 24, e.g., by an electric connection or a data connection (such as a fiber optic connection or a wireless connection). In the first processor 3, the signals are passed from the input 31 to a computation unit 32, which generates an original data set comprising content data and metadata from the signals. The original data set may then be saved in the memory 33 of the processor which is connected to the computation unit 32.

The physical measuring instrument 2 may be a scanning confocal fluorescence microscope. In this case, the light source 21 is a laser source, the illumination light I is an excitation laser beam and the physical measuring instrument 2 further comprises a scanning device (e.g., a galvanometric scanner) configured to scan the focus of the excitation laser beam through the specimen 4. Further, according to this example, the detection light D may be fluorescence light emitted by fluorophores in the specimen 4, the scanning device (not shown) may de-scan the fluorescence light, and a detection pinhole may be arranged in the detection beam path between the beam splitter 22 and the detector 24 in a confocal plane conjugated to a focal plane in the specimen 4. In case of a confocal scanning microscope, the detector 24 is typically a point detector, such as an avalanche photodiode, a photomultiplier tube or a hybrid detector. Content data obtained by such a confocal microscope setup will typically contain two-dimensional scan images formed by pixels or three-dimensional images formed by voxels, wherein the pixels or voxels are obtained by measuring the light intensity of the detection light D point by point using the detector 24 while scanning the illumination light I over the specimen 4 and de-scanning the detection light D. According to a variant of this example, an additional light source (not shown) of the microscope may generate STED light capable of causing stimulated emission depletion of the fluorophores or switching light capable of activating or de-activating the fluorophores in the specimen 4. An additional beam shaping device (such as a phase plate or a spatial light modulator) may be provided in the illumination light path to generate a focus of the STED or switching light with one or several local light intensity minima in the focal plane in the specimen 4. In this manner, the resolution may be increased beyond the diffraction limit.

Alternatively, the microscope may use widefield illumination (e.g., with excitation light) of the specimen 4 and the detector 24 may, e.g., be a camera which picks up the detection light D (e.g., fluorescence light) from a field of view as a two-dimensional image as known from the prior art. In this case, the content data will typically contain widefield images of certain areas of the specimen 4.

Yet, alternatively, the microscope 2 may be a MINFLUX microscope. Typically, according to the MINFLUX method, single emitters, e.g., fluorophores, or reflectors in the specimen 4 are localized at high precision by sequentially illuminating the specimen with an excitation light distribution comprising a central local minimum in a pattern (set of targeted coordinates, STC, or targeted coordinate pattern, TCP) and measuring the corresponding light intensities using a point detector. To this end, the illumination beam path of a MINFLUX microscope typically comprises a beam shaping device (e.g., a phase plate or a spatial light modulator) to form the excitation light distribution with the local minimum in the focal plane in the specimen 4, fast scanning devices (e.g., electro optical deflectors) and optionally an axial focus deflector, such as a deformable mirror, to move the central minimum of the light distribution to the positions of the pattern. From the light intensities corresponding to the relative pattern positions, the position of a single emitter or reflector is estimated as described in the prior art. The localization procedure is typically performed iteratively, wherein the previous position estimate is used as the center of the pattern in each iteration step.

According to related methods, the specimen may be scanned with a STED light distribution comprising a minimum and an excitation light distribution comprising an intensity maximum. By localizing several emitters or reflectors sequentially in this manner, a high-resolution image of the emitter or reflector distribution in the specimen may be generated.

Therefore, data sets generated from MINFLUX and related localization methods will typically be two-dimensional or three-dimensional images constructed from several localizations. However, a data set within the meaning of the present specification may also be constituted by localization data of a single emitter or reflector. Furthermore, the original data set may comprise or consist of localization raw data, e.g., a set of measured light intensity values allocated to respective pattern positions of the local minimum of the light intensity distribution from one or several iterations and/or one or several emitters or reflectors.

The computation unit 32 of the first processor 3 is further configured to generate a digital proof of authenticity indicating the authenticity of the original data set. The digital proof of authenticity may be, e.g., a non-fungible token (NFT) stored in a block chain or a digital signature, as described below in more detail.

Briefly, according to a first embodiment, an NFT indicating the authenticity of the original data set may be generated from the original data set by the computation unit 32 of the first processor 3 and then transferred to a block chain via the output 34 connected to the computation unit 32. The block chain may be stored, propagated and validated by a plurality of full nodes. In Fig. 1, a first computer 5, a second computer 6 and a third computer 7 forming full nodes of a distributed network 8 are schematically depicted as a simplified example. In addition, the first processor 3 itself may serve as a full node and participate in propagating and validating the block chain.

According to a second embodiment, the computation unit 32 of the first processor 3 may generate a digital signature of the original data set. This digital signature may be stored in the memory 33 and sent (particularly together with the original data set) to any of the computers 5, 6, 7 to authenticate the original data set. Computers 5, 6, 7 can then validate the digital signature as described below.

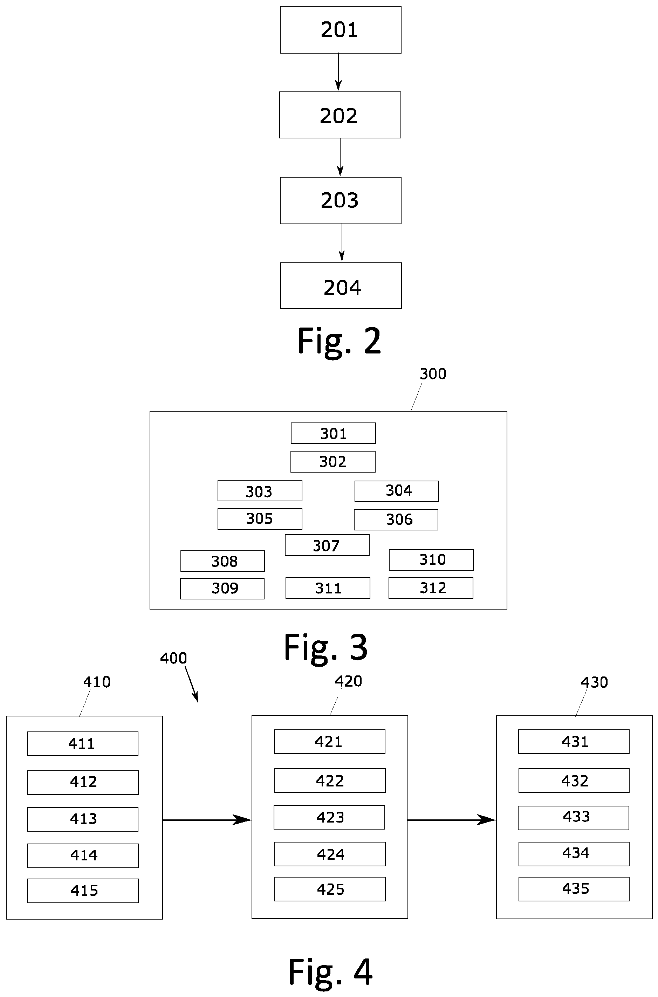

**Fig. 2** is a flowchart depicting the steps of the method according to a first embodiment of the invention, particularly using the device 1 shown in Fig. 1. According to the first embodiment, an NFT is generated as a digital proof of authenticity.

In step 201, an original data set is acquired by the physical measuring instrument 2 as described above. In particular, the physical measuring instrument 2 generates an image or several images from certain parts of the specimen 4, or one or several emitters in the specimen 4 are localized by the physical measuring instrument 2. The generated original data set particularly contains content data, e.g., images or localization data, and metadata, e.g., comprising measuring parameters used during acquisition of the images or during localization. The original data set is particularly stored in the memory 33 of the first processor 3.

According to step 202, a first private key identifying a first user of the physical measuring instrument 2 is obtained by the computation unit 32 of the first processor 3. The first private key may be stored in the memory 33 and retrieved by the computation unit 32. Alternatively, the first private key may be entered by the first user via an interface of the first processor 3. Optionally, in case the first private key is stored in the memory 33, the first user may have to identify himself/herself to trigger retrieval of the first private key, e.g., by entering a password via the interface, by connecting a digital signature card to the first processor 3 or by initiating acquisition of biometric data by the first processor 3 (e.g., via a retina scanner, a fingerprint scanner or a camera for obtaining a picture which can be analyzed by a face detection algorithm). The first private key is particularly linked to a first public key. The first public key may be allocated to a digital wallet of the first user used to store NFTs in a block chain. By means of the first private key, the digital proof of authenticity indicates who generated the original data set.

Optionally, a fourth private key identifying a second user may be obtained by the computation unit 32, in the same or a similar manner as described above for the first private key. The fourth private key may be used as an additional verification of the generated NFT and may be linked to the first public key allocated to the digital wallet of the first user or may be linked to a separate public key allocated to a separate digital wallet of the second user. The second user may be a superior of the first user who needs to verify the data set before generating the digital proof of authenticity.

Further, in step 203, a second private key identifying the physical measuring instrument 2 is obtained by the computation unit 32, e.g., from the memory 33 or from an internal memory of the physical measuring instrument 2. In particular, the second private key is stored in a manner such that the second private key is not erasable and modifiable, and optionally such that the second private key cannot be directly retrieved, except by the computation unit 32 during generation of an NFT. E.g., the second private key may be stored in the memory 33 or the internal memory of the physical measuring instrument 2 in an encrypted form. For instance, the second private key may be stored in a trusted platform module (TPM) of the physical measuring instrument 2 or first processor 3. The second private key indicates the device, which was used to generate the original data set. In particular, the second private key is linked to the first public key, i.e., to the same public key as the first private key (and particularly the same digital wallet of the first user). Optionally, a third private key identifying the specimen 4 may be obtained by the computation unit 32, e.g., based on a scan, by the physical measuring instrument 2, of a physical identifier (e.g., a number, a barcode or a QR code) inscribed in or on the specimen 4 or on a specimen carrier carrying the specimen 4.

In step 204, an NFT authenticating the original data set obtained by the physical measuring instrument 2 is generated in a block chain by the first processor 3. To this end, in particular, a transaction, e.g., a generation of a smart contract governing the NFT is initiated, and this transaction is included in a new block of the block chain, which is verified by the distributed network 8. Optionally, the NFT can later be updated, e.g., when a processed data set is generated from the original data set.

**Fig. 3** schematically shows an example of an NFT 300 which can be used to authenticate the original data set generated by the physical measuring instrument 2 and optionally the processed data set.

The NFT 300 comprises a token identifier 301, which may be a consecutive number issued to the NFTs in a particular block chain, and a time stamp 302 indicating the time of generating ('minting') the NFT 300.

Furthermore, the NFT 300 comprises a first owner identifier 303 indicating the owner or creator of the NFT 300, i.e., the first user of the physical measuring instrument 2. For instance, the first owner identifier 303 may be the first public key which is linked to the first private key of the first user. Notably, in contrast to other NFTs, e.g., those used to represent artworks or collectibles according to the prior art, ownership of the NFT 300 according to the present invention cannot be transferred, in other words the owner identifier 303 cannot be changed once the NFT 300 has been minted. Thereby, it is ensured that the NFT 300 authenticates the original creator of the data set linked to the NFT 300, as long as the NFT 300 exists in the block chain.

Optionally, the NFT 300 further comprises a second owner identifier 304, indicating the identity of, e.g., a co-creator of the dataset or a person verifying the data set before the data set is authenticated by the NFT 300, e.g., a group leader or manager. The second owner identifier 304 may be the fourth private key of the second user of the physical measuring instrument 2.

To authenticate the physical measuring instrument 2, by which the original data set was generated, the NFT 300 further comprises a first device identifier 305, e.g., a public key linked to the second private key identifying the physical measuring instrument 2.

Optionally, the NFT 300 further comprises at least one second device identifier 306 which identifies a second processor separate from the first processor 3 connected to the physical measuring instrument 2, on which a certain processed data set was generated from the original data set. This second device identifier 306 may be a public key linked to the fifth private key of the second processor.

In case the original data set or a processed data set is published, the NFT 300 may comprise a publication identifier 307, i.e., an indicator of a web address which can be used to access the published data set (with or without identification, e.g., password protection). In cases, in which the data set has not been published upon minting of the NFT 300, the publication identifier 307 may be initially left blank or assigned a value indicating that the data set has not been published yet. In particular, the publication identifier 307 may be assigned a value indicating a publication address in a later update of the NFT 300.

To represent the content data (e.g., images and/or localization data) comprised in the original data set, the NFT 300 comprises at least one first content identifier 308. The first content identifier 308 may be a hash value obtained by applying a hash function to the content data. Alternatively, e.g., the first content identifier 308 may be a hash value obtained from content data and metadata comprised in the original data set. For example, a first hash value obtained by applying a hash function to the content data may be stored in the metadata of the original data set, and the first content identifier 308 may be a second hash value obtained by applying a hash function to the metadata. In case the content data comprise more than one subset of data (e.g., several images constituting a movie or a z-stack), a separate first content identifier 308 may be assigned to each subset, or a single first content identifier 308 may be generated from the subsets, e.g., by applying a hash function to the total content data.

Furthermore, the NFT 300 may comprise at least one first metadata identifier 309 representing metadata of the original data set. For example, the metadata identifier 309 may be a hash value obtained from the metadata of the original data set.

When a processed data set is generated from the original data set, the NFT 300 may be updated by a transaction in the block chain. In particular, during each update, a second content identifier 310 representing the processed content data of the processed data set is added to the NFT 300. The second content identifier 310 may be a hash value obtained by applying a hash function to the processed content data and/or metadata in the same or a similar manner as explained above for the original content data and the first content identifier 308. Each second content identifier 310 may comprise a time stamp indicating the time at which the NFT 300 was updated. Furthermore, during an update, a second metadata identifier 311 representing metadata describing processing parameters used during processing of the original data set (or further processing of a processed data set) may be added to the NFT 300. Each second metadata identifier 311 may be linked to a respective second content identifier 310 to indicate which processed content was generated using the processing parameters represented by the respective second metadata identifier 311.

Optionally, the NFT 300 may comprise a unique specimen identifier 312 identifying the specimen 4 from which the original data have been obtained. The specimen identifier 312 may be derived from a physical identifier (e.g., a number a barcode or a QR code) inscribed on the specimen 4 or on a specimen carrier (such as a microscope slide or a cover slip).

**Fig. 4** schematically shows an example of a block chain 400 used to store the NFT 300 shown in Fig. 3. The block chain comprises a plurality of blocks (three blocks 410, 420, 430 are depicted for simplicity, although an actual block chain may contain many thousands of blocks). Each block 410, 420, 430 comprises a block header 411, 421, 431, a time stamp 412, 422, 432 indicating the time at which the respective block 410, 420, 430 was generated, a previous hash value 413, 423, 433 obtained by applying a hash function to data included in the previous block 410, 420, 430, a Merkle root 414, 415, 416 which is a root of a Merkle tree, the root being a hash value obtained by successive application of a hash function to the leaves of the Merkle tree indicating transactions to be verified by the current block 410, 420, 430, and optionally, a nonce 415, 425, 435 which is a random number applied in an exemplary proof-of-work consensus mechanism of the block chain 400 as known from the prior art.

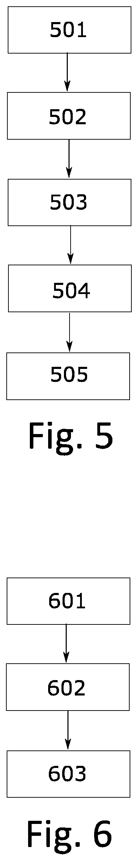

**Fig. 5** is a flowchart describing steps of the method according to a second embodiment of the invention, wherein the digital proof of authenticity is a digital signature.

According to step 501, an original data set is acquired by the device 1 using the physical measuring instrument 2 as described above.

In step 502, a first private key of the first user is obtained, e.g., in a similar manner as described above for the first embodiment. The first private key is linked to a first public key.

Step 503 consists of obtaining a second private key of the physical measuring instrument 2, e.g., in a similar manner as described above for the first embodiment. The second private key is linked to the first public key, i.e., to the same public key as the first private key, or the second private key is linked to a second public key.

According to step 504, a digital signature is generated from the original data set, particularly the content data and/or the metadata, the first private key and/or the second private key. For instance, the digital signature may be a hash value obtained by applying a hash function to the content data, the metadata, the first private key and/or the second private key. In the same manner, a digital signature of a processed data set obtained from the original data set may be generated, optionally using only the first private key or the first private key and a fifth private key of a second processor used to process the data set. If a second user is involved in the authentication process as described above, a fourth private key identifying the second user may also be used to generate the digital signature. This fourth private key may be linked to the first public key, or to a separate third public key.

In step 505, a message comprising the original data set and/or the processed data set together with the respective digital signature and optionally, the first, second and/or fourth public key, is sent.

**Fig. 6** is a further flow chart illustrating a verification procedure of the digital signature used in the second embodiment of the invention.

In step 601, the message comprising the original data set and/or the processed data set together with the respective digital signature or signatures is received.

According to step 602, one or several public key(s) is/are received, e.g., together with the message of step 601 or in a separate message.

Finally, step 603 consists of verifying the digital signature using the public key or public keys, e.g., by receiving a digital certificate from a trusted agency indicating that the public key or keys is / are linked to the first user, the second user, the physical measuring instrument 2 and/or the second processor and verifying that the digital signature was generated using the private key(s) linked to the respective public key(s).

### LIST OF REFERENCE SIGNS

- 1: Device for authenticating a data set
- 2: Physical measuring instrument
- 3: First processor
- 4: Specimen
- 5: First computer
- 6: Second computer
- 7: Third computer
- 8: Distributed network
- 21: Light source
- 22: Beam splitter
- 23: Objective
- 24: Detector
- 25: Specimen holder
- 31: Input
- 32: Computation unit
- 33: Memory
- 34: Output
- 300: Non-fungible token
- 301: Token identifier
- 302: Time stamp of NFT
- 303: First owner identifier
- 304: Second owner identifier
- 305: First device identifier
- 306: Second device identifier
- 307: Publication identifier
- 308: First content identifier
- 309: First metadata identifier
- 310: Second content identifier
- 311: Second metadata identifier
- 312: Specimen identifier
- 400: Block chain
- 410,420,430: Block
- 411,421,431: Block header
- 412,422,432: Time stamp of block
- 413,423,433: Previous hash value
- 414,424,434: Merkle root
- 415,425,435: Nonce

## Claims

1. A method for authenticating a data set, wherein an original data set is acquired from a specimen (4) held by a specimen holder (25) using a physical measuring instrument (2), particularly a microscope or a tomograph, wherein a digital proof of authenticity indicating the authenticity of the original data set is generated by a first processor (3) connected to or comprised in the physical measuring instrument (2), wherein the digital proof of authenticity is a non-fungible token (300) stored in a distributed ledger, wherein the non-fungible token (300) comprises a unique first device identifier (305) indicating the physical measuring instrument (2),
wherein the non-fungible token (300) comprises a unique first owner identifier (303) indicating a first user of the physical measuring instrument (2).

2. The method according to claim 1, wherein the distributed ledger is a block chain (400).

3. The method according to claim 1 or 2, wherein the non-fungible token (300) comprises a first content identifier (308) identifying or encoding at least a part of the original data set, wherein particularly the first content identifier (308) is a hash value obtained by applying a hash function to the original data set.

4. The method according to one of the preceding claims, wherein the non-fungible token (300) comprises a unique specimen identifier (312) indicating the specimen (4).

5. The method according to one of the preceding claims, wherein the first owner identifier (303) and/or the first device identifier (305) and/or the specimen identifier (312) is stored in a non-erasable and non-editable manner in the distributed ledger, particularly the block chain (400).

6. The method according to one of the preceding claims, wherein the method comprises obtaining a first private key associated with a first user and/or a second private key associated with the physical measuring instrument (2) and/or a third private key associated with the specimen (4), wherein particularly the digital proof of authenticity is generated using the first private key and/or the second private key and/or the third private key, wherein particularly the digital signature is generated using the first private key, the second private key and/or the third private key, and a hash value obtained by applying a hash function to the original data set.

7. The method according to one of the preceding claims, wherein at least one processed data set is generated from the original data set, wherein the digital proof of authenticity is updated, such that the digital proof of authenticity indicates the authenticity of the original data set and the at least one processed data set, wherein particularly
- the non-fungible token (300) further comprises at least one second content identifier (310) identifying or encoding the processed data set, wherein more particularly the second content identifier (310) is a hash value obtained by applying a hash function to the original data set and the processed data set,
and/or
- the hash function is applied to the original data set and the processed data set to generate a combined hash value, wherein the digital proof of authenticity is updated using the combined hash value, wherein more particularly the combined hash value is generated by obtaining a first hash value by applying the hash function to the original data set, and obtaining a second hash value by applying the hash function to the first hash value and the processed data set.

8. The method according to one of the claims 1 to 7, wherein the digital proof of authenticity is automatically generated by the first processor (3) in response to the original data set being acquired, wherein particularly the original data set comprises metadata indicating at least one measuring parameter of the physical measuring instrument (2) used during acquisition of the original data set, and wherein at least a part of the metadata are automatically selected by the first processor (3), wherein the digital proof of authenticity indicates the authenticity of the selected metadata.

9. The method according to one of the claims 1 to 7, wherein the digital proof of authenticity is generated by the first processor (3) for the acquired original data set in response to a user command received by the first processor (3), wherein particularly the original data set comprises metadata indicating at least one measuring parameter of the physical measuring instrument (2) used during acquisition of the original data set, and wherein at least a part of the metadata is selected, wherein the digital proof of authenticity indicates the authenticity of the selected metadata, and wherein the metadata are automatically selected by the first processor (3), or the metadata are selected based on a user selection received by the first processor (3).

10. The method according to one of the preceding claims, wherein the original data set and/or the processed data set is uploaded from the first processor (3) to a publication server to publish the original data set and/or the processed data set together with the digital proof of authenticity.

11. A device (1) for authenticating a data set by the method according to one of the claims 1 to 10, wherein the device (1) comprises a physical measuring instrument (2), particularly a microscope or a tomograph, and a specimen holder (25) configured to hold a specimen (4), wherein the device (1) is configured to acquire an original data set from the specimen (4) held by the specimen holder (25),
wherein the device (1) comprises a processor (3) configured to generate a digital proof of authenticity indicating the authenticity of the original data set, wherein the digital proof of authenticity is a non-fungible token (300) stored in a distributed ledger, and wherein the non-fungible token (300) comprises a unique first owner identifier (303) indicating a first user of the physical measuring instrument (2) and the non-fungible token (300) comprises a unique first device identifier (305) indicating the physical measuring instrument (2).

12. The device (1) according to claim 11, wherein the device (1), particularly the physical measuring instrument (2) or the first processor (3), comprises a trusted platform module, wherein particularly the second private key associated with the physical measuring instrument (2) is stored on the trusted platform module, wherein more particularly the trusted platform module is configured to generate a hash value by applying a hash function at least to the second private key and/or generate the digital proof of authenticity.

13. A computer program comprising instructions configured to cause the device (1) according to claim 11 or 12 to execute the method according to one of the claims 1 to 10.

## Patentansprüche

1. Verfahren zur Authentifizierung eines Datensatzes, wobei ein Originaldatensatz von einer Probe (4), die von einem Probenhalter (25) gehalten wird, unter Verwendung eines physikalischen Messinstruments (2), insbesondere eines Mikroskops oder eines Tomographen, erfasst wird, wobei ein digitaler Echtheitsnachweis, der die Echtheit des Originaldatensatzes angibt, von einem ersten Prozessor (3) erzeugt wird, der mit dem physikalischen Messinstrument (2) verbunden ist oder darin enthalten ist, wobei der digitale Echtheitsnachweis ein NFT (300) ist, der in einem Distributed-Ledger gespeichert ist, wobei der NFT (300) eine eindeutige erste Gerätekennung (305) umfasst, die das physikalische Messinstrument (2) angibt, wobei der NFT (300) eine eindeutige erste Eigentümerkennung (303) umfasst, die einen ersten Benutzer des physikalischen Messinstruments (2) angibt.

2. Verfahren nach Anspruch 1, wobei das Distributed-Ledger eine Blockchain (400) ist.

3. Verfahren nach Anspruch 1 oder 2, wobei der NFT (300) eine erste Inhaltskennung (308) umfasst, die mindestens einen Teil des Originaldatensatzes identifiziert oder kodiert, insbesondere wobei die erste Inhaltskennung (308) ein Hashwert ist, der durch Anwendung einer Hashfunktion auf den Originaldatensatz erhalten wird.

4. Verfahren nach einem der vorhergehenden Ansprüche, wobei der NFT (300) eine eindeutige Probenkennung (312) umfasst, die die Probe (4) identifiziert.

5. Verfahren nach einem der vorhergehenden Ansprüche, wobei die erste Eigentümerkennung (303) und/oder die erste Gerätekennung (305) und/oder die Probenkennung (312) in einer nicht löschbaren und nicht editierbaren Weise in dem Distributed-Ledger, insbesondere der Blockchain (400), gespeichert ist.

6. Verfahren nach einem der vorhergehenden Ansprüche, wobei das Verfahren das Erhalten eines ersten privaten Schlüssels, der einem ersten Benutzer zugeordnet ist, und/oder eines zweiten privaten Schlüssels, der dem physikalischen Messinstrument (2) zugeordnet ist, und/oder eines dritten privaten Schlüssels, der der Probe (4) zugeordnet ist, umfasst, insbesondere wobei der digitale Echtheitsnachweis unter Verwendung des ersten privaten Schlüssels und/oder des zweiten privaten Schlüssels und/oder des dritten privaten Schlüssels erzeugt wird, insbesondere wobei die digitale Signatur unter Verwendung des ersten privaten Schlüssels, des zweiten privaten Schlüssels und/oder des dritten privaten Schlüssels und eines Hashwerts, der durch Anwendung einer Hashfunktion auf den Originaldatensatz erhalten wird, erzeugt wird.

7. Verfahren nach einem der vorhergehenden Ansprüche, wobei mindestens ein verarbeiteter Datensatz aus dem Originaldatensatz erzeugt wird, wobei der digitale Echtheitsnachweis aktualisiert wird, so dass der digitale Echtheitsnachweis die Echtheit des Originaldatensatzes und des mindestens einen verarbeiteten Datensatzes angibt, wobei insbesondere
- der NFT (300) ferner mindestens eine zweite Inhaltskennung (310) umfasst, die den verarbeiteten Datensatz identifiziert oder kodiert, insbesondere wobei die zweite Inhaltskennung (310) ein Hashwert ist, der durch Anwendung einer Hashfunktion auf den Originaldatensatz und den verarbeiteten Datensatz erhalten wird,
und/oder
- die Hash-Funktion auf den Originaldatensatz und den verarbeiteten Datensatz angewendet wird, um einen kombinierten Hash-Wert zu erzeugen, wobei der digitale Echtheitsnachweis unter Verwendung des kombinierten Hash-Werts aktualisiert wird, insbesondere wobei der kombinierte Hash-Wert erzeugt wird, indem ein erster Hash-Wert durch Anwenden der Hash-Funktion auf den Originaldatensatz und ein zweiter Hash-Wert durch Anwenden der Hash-Funktion auf den ersten Hash-Wert und den verarbeiteten Datensatz erhalten wird.

8. Verfahren nach einem der Ansprüche 1 bis 7, wobei der digitale Echtheitsnachweis automatisch von dem ersten Prozessor (3) als Reaktion auf die Erfassung des Originaldatensatzes erzeugt wird, insbesondere wobei der Originaldatensatz Metadaten umfasst, die mindestens einen Messparameter des physikalischen Messinstruments (2) angeben, das während der Erfassung des Originaldatensatzes verwendet wurde, und wobei mindestens ein Teil der Metadaten automatisch von dem ersten Prozessor (3) ausgewählt wird, wobei der digitale Echtheitsnachweis die Echtheit der ausgewählten Metadaten angibt.

9. Verfahren nach einem der Ansprüche 1 bis 7, wobei der digitale Echtheitsnachweis von dem ersten Prozessor (3) für den erfassten Originaldatensatz als Reaktion auf einen von dem ersten Prozessor (3) empfangenen Nutzerbefehl erzeugt wird, insbesondere wobei der Originaldatensatz Metadaten umfasst, die mindestens einen Messparameter des physikalischen Messinstruments (2) angeben, der während der Erfassung des Originaldatensatzes verwendet wurde, und wobei mindestens ein Teil der Metadaten ausgewählt wird, wobei der digitale Echtheitsnachweis die Echtheit der ausgewählten Metadaten angibt und wobei die Metadaten automatisch von dem ersten Prozessor (3) ausgewählt werden oder die Metadaten auf der Grundlage einer von dem ersten Prozessor (3) empfangenen Benutzerauswahl ausgewählt werden.

10. Verfahren nach einem der vorhergehenden Ansprüche, wobei der Originaldatensatz und/oder der verarbeitete Datensatz von dem ersten Prozessor (3) auf einen Veröffentlichungsserver hochgeladen wird, um den Originaldatensatz und/oder den verarbeiteten Datensatz zusammen mit dem digitalen Echtheitsnachweis zu veröffentlichen.

11. Vorrichtung (1) zur Authentifizierung eines Datensatzes nach dem Verfahren gemäß einem der Ansprüche 1 bis 10, wobei die Vorrichtung (1) ein physikalisches Messinstrument (2), insbesondere ein Mikroskop oder einen Tomographen, und einen Probenhalter (25), der zum Halten einer Probe (4) ausgebildet ist, umfasst, wobei die Vorrichtung (1) dazu ausgebildet ist, einen Originaldatensatz von der von dem Probenhalter (25) gehaltenen Probe (4) zu erfassen,
wobei die Vorrichtung (1) einen Prozessor (3) umfasst, der dazu ausgebildet ist, einen digitalen Echtheitsnachweis zu erzeugen, der die Echtheit des Originaldatensatzes angibt, wobei der digitale Echtheitsnachweis ein NFT (300) ist, der in einem Distributed-Ledger gespeichert ist, und wobei der NFT (300) eine eindeutige erste Eigentümerkennung (303) umfasst, die einen ersten Benutzer des physikalischen Messinstruments (2) angibt, und der NFT (300) eine eindeutige erste Gerätekennung (305) umfasst, die das physikalische Messinstrument (2) angibt.

12. Vorrichtung (1) gemäß Anspruch 11, wobei die Vorrichtung (1), insbesondere das physikalische Messinstrument (2) oder der erste Prozessor (3), ein Trusted-Platform-Modul umfasst, insbesondere wobei der zweite private Schlüssel, der dem physikalischen Messinstrument (2) zugeordnet ist, auf dem Trusted-Platform-Modul gespeichert ist, insbesondere wobei das Trusted-Platform-Module dazu ausgebildet ist, durch Anwenden einer Hashfunktion zumindest auf den zweiten privaten Schlüssel einen Hashwert zu erzeugen und/oder den digitalen Echtheitsnachweis zu erzeugen.

13. Computerprogramm, umfassend Befehle, die dazu ausgebildet sind, die Vorrichtung (1) gemäß Anspruch 11 oder 12 dazu zu veranlassen, das Verfahren gemäß einem der Ansprüche 1 bis 10 auszuführen.

## Revendications

1. Procédé d'authentification d'un ensemble de données, dans lequel un ensemble de données original est acquis à partir d'un échantillon (4) maintenu par un support d'échantillon (25) à l'aide d'un instrument de mesure physique (2), en particulier un microscope ou un tomographe, dans lequel une preuve d'authenticité numérique indiquant l'authenticité de l'ensemble de données original est générée par un premier processeur (3) connecté à ou compris dans l'instrument de mesure physique (2), dans lequel la preuve d'authenticité numérique est un NFT (300) stocké dans un registre distribué, dans lequel le NFT (300) comprend un premier identifiant de dispositif (305) unique indiquant l'instrument de mesure physique (2), dans lequel le NFT (300) comprend un premier identifiant de propriétaire (303) unique indiquant un premier utilisateur de l'instrument de mesure physique (2).

2. Procédé selon la revendication 1, dans lequel le registre distribué est une chaîne de blocs (400).

3. Procédé selon la revendication 1 ou 2, dans lequel le NFT (300) comprend un premier identifiant de contenu (308) identifiant ou codant au moins une partie de l'ensemble de données original, dans lequel en particulier le premier identifiant de contenu (308) est une valeur de hachage obtenue en appliquant une fonction de hachage à l'ensemble de données original.

4. Procédé selon l'une des revendications précédentes, dans lequel le NFT (300) comprend un identifiant d'échantillon (312) unique indiquant l'échantillon (4).

5. Procédé selon l'une des revendications précédentes, dans lequel le premier identifiant de propriétaire (303) et/ou le premier identifiant de dispositif (305) et/ou l'identifiant d'échantillon (312) est stocké de manière non effaçable et non modifiable dans le registre distribué, en particulier la chaîne de blocs (400).

6. Procédé selon l'une des revendications précédentes, dans lequel le procédé comprend l'obtention d'une première clé privée associée à un premier utilisateur et/ou d'une deuxième clé privée associée à l'instrument de mesure physique (2) et/ou d'une troisième clé privée associée à l'échantillon (4), dans lequel, en particulier, la preuve d'authenticité numérique est générée à l'aide de la première clé privée et/ou de la deuxième clé privée et/ou de la troisième clé privée, dans lequel, en particulier, la signature numérique est générée à l'aide de la première clé privée, la deuxième clé privée et/ou la troisième clé privée, et une valeur de hachage obtenue en appliquant une fonction de hachage à l'ensemble de données original.

7. Procédé selon l'une des revendications précédentes, dans lequel au moins un ensemble de données traité est généré à partir de l'ensemble de données original, dans lequel la preuve d'authenticité numérique est mise à jour, de telle sorte que la preuve d'authenticité numérique indique l'authenticité de l'ensemble de données original et de l'au moins un ensemble de données traité, dans lequel en particulier
- le NFT (300) comprend en outre au moins un deuxième identifiant de contenu (310) identifiant ou codant l'ensemble de données traité, dans lequel plus particulièrement le deuxième identifiant de contenu (310) est une valeur de hachage obtenue en appliquant une fonction de hachage à l'ensemble de données original et à l'ensemble de données traité,
et/ou
- la fonction de hachage est appliquée à l'ensemble de données original et à l'ensemble de données traité pour générer une valeur de hachage combinée, dans lequel la preuve d'authenticité numérique est mise à jour à l'aide de la valeur de hachage combinée, dans lequel plus particulièrement la valeur de hachage combinée est générée en obtenant une première valeur de hachage en appliquant la fonction de hachage à l'ensemble de données d'origine, et en obtenant une deuxième valeur de hachage en appliquant la fonction de hachage à la première valeur de hachage et à l'ensemble de données traité.

8. Procédé selon l'une des revendications 1 à 7, dans lequel la preuve d'authenticité numérique est générée automatiquement par le premier processeur (3) en réponse à l'acquisition de l'ensemble de données original, dans lequel en particulier l'ensemble de données original comprend des métadonnées indiquant au moins un paramètre de mesure de l'instrument de mesure physique (2) utilisé pendant l'acquisition de l'ensemble de données original, et dans lequel au moins une partie des métadonnées est sélectionnée automatiquement par le premier processeur (3), dans lequel la preuve d'authenticité numérique indique l'authenticité des métadonnées sélectionnées.

9. Procédé selon l'une des revendications 1 à 7, dans lequel la preuve d'authenticité numérique est générée par le premier processeur (3) pour l'ensemble de données original acquis en réponse à une commande utilisateur reçue par le premier processeur (3), dans lequel en particulier l'ensemble de données original comprend des métadonnées indiquant au moins un paramètre de mesure de l'instrument de mesure physique (2) utilisé pendant l'acquisition de l'ensemble de données original, et dans lequel au moins une partie des métadonnées est sélectionnée, dans lequel la preuve d'authenticité numérique indique l'authenticité des métadonnées sélectionnées, et dans lequel les métadonnées sont automatiquement sélectionnées par le premier processeur (3), ou les métadonnées sont sélectionnées sur la base d'une sélection utilisateur reçue par le premier processeur (3).

10. Procédé selon l'une des revendications précédentes, dans lequel l'ensemble de données original et/ou l'ensemble de données traité est téléchargé depuis le premier processeur (3) vers un serveur de publication afin de publier l'ensemble de données original et/ou l'ensemble de données traité avec la preuve d'authenticité numérique.

11. Dispositif (1) pour authentifier un ensemble de données selon le procédé selon l'une des revendications 1 à 10, dans lequel le dispositif (1) comprend un instrument de mesure physique (2), en particulier un microscope ou un tomographe, et un support d'échantillon (25) configuré pour maintenir un échantillon (4), dans lequel le dispositif (1) est configuré pour acquérir un ensemble de données original à partir de l'échantillon (4) maintenu par le support d'échantillon (25),
dans lequel le dispositif (1) comprend un processeur (3) configuré pour générer une preuve d'authenticité numérique indiquant l'authenticité de l'ensemble de données original, dans lequel la preuve d'authenticité numérique est un NFT (300) stocké dans un registre distribué, et dans lequel le NFT (300) comprend un premier identifiant de propriétaire (303) unique indiquant un premier utilisateur de l'instrument de mesure physique (2) et le NFT (300) comprend un premier identifiant de dispositif (305) unique indiquant l'instrument de mesure physique (2).

12. Le dispositif (1) selon la revendication 11, dans lequel le dispositif (1), en particulier l'instrument de mesure physique (2) ou le premier processeur (3), comprend un module de plateforme sécurisée, dans lequel en particulier la deuxième clé privée associée à l'instrument de mesure physique (2) est stockée sur le module de plateforme sécurisée, dans lequel plus particulièrement le module de plateforme sécurisée est configuré pour générer une valeur de hachage en appliquant une fonction de hachage au moins à la deuxième clé privée et/ou générer la preuve numérique d'authenticité.

13. Programme informatique comprenant des instructions configurées pour amener le dispositif (1) selon la revendication 11 ou 12 à exécuter le procédé selon l'une des revendications 1 à 10.
